# EUROPEAN PATENT SPECIFICATION

(11) **EP 1 504 747 B1**
(45) Date of publication and mention of the grant of the patent: **19.10.2005**
(21) Application number: 03018083.0
(22) Date of filing: 08.08.2003
(51) Int. Cl.: A61K 7/06

(54) **Cosmetic composition for hair comprising cetyl PEG/PPG-10/1 dimethicone and ceramide**
Kosmetische Haarzusammensetzung enthaltend Cetyl PEG/PPG-10/1 Dimethicon und ein Ceramid
Composition cosmétique capillaire comprenant une cétyl PEG/PPG-10/1 diméthicone et une céramide

(43) Date of publication of application: 09.02.2005
(73) Proprietor: KPSS-Kao Professional Salon Services GmbH, 64297 Darmstadt (DE)
(72) Inventor: Grit, Mustafa, Dr., 64579 Gernsheim (DE); Molenda, Michael, 60598 Frankfurt (DE); Hoffmann, Martin, 64673 Zwingenberg (DE)

(56) References cited:
- DE-C- 19 729 080
- US-A- 5 560 917
- US-A- 5 776 480
- US-A- 5 889 047

## Description

The present invention concerns cosmetic composition which shows optimum conditioning and repair properties for human hair comprising hair care and repair agents.

Hair conditioning agents and compositions including those have been known for years and have proven very successful on the market. In addition hair repair benefits have also been claimed successfully previously.

Conditioning and hair repair ingredients have been disclosed in the state of the art. Those are usually in shampoo and conditioner pair with or without any leave - in products and comprising either in surfactant base or in an emulsion base those ingredients.

Although these products are proven as such, it is still desirable to improve their efficiency, in particular with regard to volume, gloss, combability and body of the hair washed and treated with these products. In addition especially for chemically damaged hair, and especially due to multiple processing with coloration, bleaching and/or waving, repair benefits are very much desirable in order to achieve hair quality towards healthy hair. This is as well desirable for healthy, undamaged hair for maintaining its good condition.

It has now been found that compositions comprising combination of cetyl PEG/PPG-10/1 dimethicone and ceramide type of ingredient gives human hair treated with these compositions benefits such as volume, gloss, combability and body as well and with a greater extent repair effect. The repair effect is especially become obvious after multiple treatments. It has further been found out it is even favorable to combine the hair repairing ceramide with fatty acids and sterol like molecules, namely phytosterols, in order to achieve advanced repair effects.

For example, DE 197 24 280 A1 discloses hair treatment prepartions comprising fatty acids and ceramide combined with conventional conditioners such as cationic surfactants for improvement of hair quality. In the document nothing is said about the combination with cetyl PEG/PPG-10/1 dimethicone with ceramide for achieving better conditioning and at the same time repair benefits.

Cetyl PEG/PPG-10/1 dimethicone has been disclosed as conditioning agent in emulsion type preparations suitable especially for skin care. EP 836 848 A2 describes cosmetic skin browning and light protection agent comprising cetyl PEG/PPG-10/1 dimethicone. Furthermore, DE 101 44 235 disclosing water in silicone (W/S) emulsions comprising cetyl PEG/PPG-10/1 dimethicone. In both documents it is disclosed that those preparations are only suitable for use on skin.

Cetyl PEG/PPG-10/1 dimethicone used in the compositions of the present invention is known with its trade name Abil^{R} EM 90. Chemically, it is a siloxane modified with polyether and alkyl groups. It has been disclosed to an emulsifier with an HLB value of 5, so that suitable as emulsifier for W/O emulsions or multiple emulsions of W/O/W or O/W/O.

In the prior art so far know, compositions are not disclosed suitable for conditioning and hair repair effects comprising at least one ceramide type of compound and cetyl PEG/PPG-10/1 dimethicone.

Cosmetic compositions for hair according to the present invention comprises ceramide of the following formula 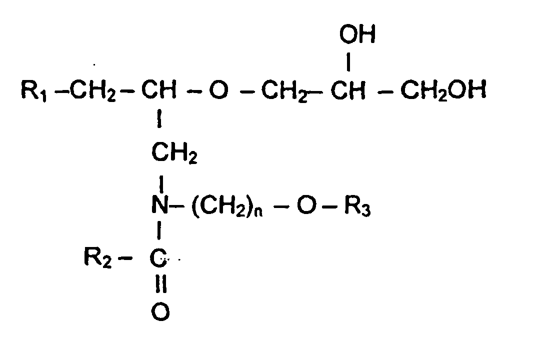 where R¹ and R² are independent from each other alkyl- or alkenyl group mit 10 to 22 carbon atoms, R³ is methyl, ethyl, n-propyl or isopropyl group and n is a number between 1 to 6, preferably 2 or 3 and cetyl PEG/PPG-10/1 dimethicone.

The combinations of the care and repair ingredients can be formulated into shampoo, conditioner or in any form of hair care formulations. Especially the conditioners are suitable for rinse off as well as leave in applications.

Shampoo compositions comprising the care and repair ingredients should show as well good shampooing properties. This is usually measured with the foaming performance while washing hair. Surfactants suitable for achieving good lathering properties according to the invention are chosen from anionic, non-ionic and zwitterionic or amphoteric ones. It is preferred that the main surfactant is anionic one and the non-ionic and zwitterionic or amphoteric surfactants are used as co-surfactants for improving foam and/or conditioning properties. Total surfactant concentration in shampoo is in the range of 1 to 50%, preferably 5 to 40% and more preferably 5 to 30% and most preferably 5 to 25% by weight calculated to the total composition.

It should be noted that the compositions according to the invention can comprise conditioning agents other than cetyl PEG/PPG-10/1 dimethicone and ceramide type of ingredients such as cationic surfactants, cationic polymers, silicone oils either non-ionic or cationic, triglycerides. The details of the other conditioning agents can be found below in the description.

Suitable anionic surfactants within the scope of the invention are contained in an amount ranging from at least 1 to 40%, preferably 5 to 30%, more preferably 5 to 25% by weight calculated to total composition. These surfactants are of the sulfate, sulfonate, carboxylate and alkyl phosphate type, especially, of course, those customarily used in shampoos, for example, the known C₁₀-C₁₈-alkyl sulfates and in particular the respective ether sulfates, for example, C₁₂-C₁₄-alkyl ether sulfate, lauryl ether sulfate, in particular with 1 to 4 ethylene oxide groups in the molecule, furthermore monoglyceride(ether)sulfates, fatty acid amide sulfates, obtained by ethoxylation and subsequent sulfatization of fatty acid alkanolamides, and the alkali salts thereof, as well as salts of long-chain mono- and dialkyl phosphates, which constitute mild, skin-compatible detergents.

Further anionic surfactants suitable within the scope of the invention are α-olefine sulfonates or the salts thereof, and in particular alkali salts of sulfosuccinic acid semiesters, for example, the disodium salt of monooctyl sulfosuccinate and the alkali salts of long-chain monoalkyl ethoxy sulfosuccinates.

Suitable surfactants of the carboxylate type are alkyl polyether carboxylic acids and the salts thereof of the formula

R₄ - (C₂H₄O)ₙ - O - CH₂COOX,

wherein R₄ is a C₈-C₂₀-alkyl group, preferably a C₁₂-C₁₄-alkyl group, n is a number from 1 to 20, preferably 2 to 17, and X is H or preferably a cation of the group sodium, potassium, magnesium, ammonium and mono or diethanol amine.

Alkyl amido polyether carboxylic acids of the general formula 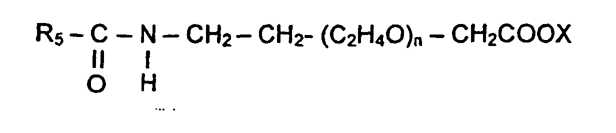 wherein R₅ is a C₈-C₂₀-alkyl group, preferably a C₁₂-C₁₄-alkyl group, n is in particular a number from 1 to 10, preferably 2.5 to 5 and X has the above-named meaning.

Such products have been known and on the market for some time, for example, under the trade name "AKYPO®" and "AKYPO-SOFT®".

C₈-C₂₀-acyl isethionates can also be used alone or in admixture with other surfactants, as well as sulfofatty acids and the esters thereof.

Useful are also mixtures of several anionic surfactants, for example, a mixture of an α- olefine sulfonate and a sulfosuccinate, or an ether sulfate and a polyether carboxylic acid or alkyl amidoether carboxylic acid.

An overview of anionic surfactants used in liquid body and/or hair cleansing products can, moreover, be found in the monography of K.Schrader, "Grundlagen und Rezepturen der Kosmetika", 2nd Ed., (1989, Hüthig Buchverlag), pp. 683 to 691.

The preferred anionic surfactant is ethoxylated fatty alcohol sulfates. The preferred quantities of anionic surfactants according to the invention are in the range of 1 to 40%, in particular of 5 to 30%, especially of 5 to 25% by weight, calculated to the total composition.

In addition to the anionic surfactants, the compositions according to the present invention comprise nonionic and amphoteric or zwitterionic surfactants. The non-ionic and amphoteric or zwitterionic surfactants can certainly be part of the preparations other than shampoos as well. Therefore the following is valid for any type of preparation comprising the cetyl PEG/PPG-10/1 dimethicone and ceramide type of ingredients.

Nonionic surfactants suitable are compounds from the category of alkyl polyglucosides with the general formula

R₆-O-(CH₂CH₂O)ₙ-Zₓ,

wherein R₆ is an alkyl group with 8 to 20, preferably 10 to 14 carbon atoms, Zₓ is a saccharide group with 5 to 6 carbon atoms, n stands for a number from 0 to 10, and x is a number between 1 and 5, preferably 1.1 to 2.5.

Further nonionic surfactants useful in the compositions according to invention are C₁₀-C₂₂-fatty alcohol ethoxylates. Especially suited C₁₀-C₂₂-fatty alcohol ethers are the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": Fatty alcohol ethoxylates are as well suitable emulsifiers.

The average degree of ethoxylation thereby ranges between about 2.5 and about 25, preferably about 10 and about 20.

Other additionally useful surfactants are, for example, the various sorbitan esters, such as polyethylene glycol sorbitan stearic acid ester, fatty acid polyglycol ester or also mixed condensates of ethylene oxide and propylene oxide, as they are on the market, for example, under the trade name "Pluronics^{R}".

Further useful nonionic surfactants are amineoxides. Such amineoxides are for example C₁₂-C₁₈- alkyl dimethyl amineoxides such as lauryl dimethyl amineoxide, C₁₂-C₁₈- alkyl amidopropyl or ethyl amineoxides, C₁₂-C₁₈ -alkyl di(hydroxyethyl) or (hydroxypropyl) amineoxides, or also amineoxides with ethylene oxide and/or propylene oxide groups in the alkyl chain.

Suitable amineoxides are on the market, for example, under the trade names "Ammonyx®", "Aromox®" or "Genaminox®".

Further nonionic surfactant components are fatty acid mono- and dialkanolamides, such as coco fatty acid monoethanolamide and myristic fatty acid monoisopropanolamide.

The preferred nonionic surfactant is of type alkyl polyglucoside. The concentration of nonionic surfactants is typically from 0.5 to 15%, preferably 0.5 - 10%, more preferably 1 - 5% by weight, calculated to the total composition.

Composition of the present invention can contain amphoteric or zwitterionic surfactants as co-surfactants in order to improve foam speed and properties such as creaminess in shampoos. The role of the amphoteric or zwitterionic surfactants can be different in especially conditioner preparations. Suitable ones are in particular the various known betaines such as fatty acid amidoalkyl betaines and sulfobetaines; for example lauryl hydroxy sulfobetaine; long-chain alkyl amino acids, such as cocoaminoacetate, cocoaminopropionate and sodium cocoamphopropionate and -acetate have also proven suitable.

In detail it is possible to use betaines of the structure 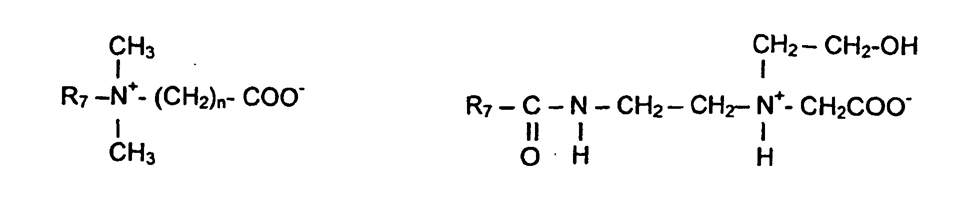 wherein R₇ is a C₈-C₁₈-alkyl group and n is 1 to 3,
sulfobetaines of the structure 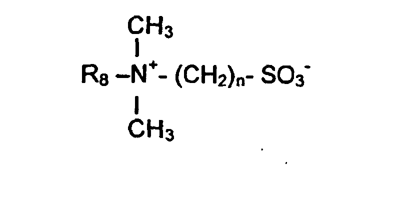 wherein R₈ is a C₈-C₁₈-alkyl group and n is 1 to 3,
and amidoalkyl betaines of the structure 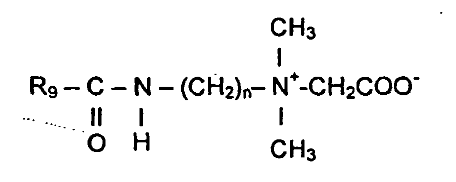 wherein R₉ is a C₈-C₁₈-alkyl group and n is 1 to 3,

Preferred are fatty acid amidoalkyl betaines, in particular cocoamidopropyl betaine, and cocoamphoacetate and -propionate, in particular the sodium salts thereof.

Preferred amphoteric or zwitterionic surfactant is those of betaine derivatives.The concentration of amphoteric or zwitterionic surfactants is from 0.1 to 15%, preferably 0.5 - 10%, more preferably 0.5 - 8% by weight, calculated to the total composition.

The weight ratio of the main surfactant to co-surfactants, being nonionic and amphoteric or zwitterionic surfactants, should be in the range of 10:1 to 1:3, preferably 5:1 to 1:2 in shampoo compositions. Furthermore, the ratio of the anionic surfactant to amphoteric or nonionic surfactants should be in the range of 10:1 to 1:1, preferably 5:1 to 2:1. These ratios are especially important for shampoo compositions, and other preparations does not have to fulfill the requirement.

Concentration of the conditioning ingredient cetyl PEG/PPG-10/1 dimethicone is in the range of 0.01 - 5%, preferably 0.01 - 4% and more preferably 0.05 - 2.5% by weight, calculated to the total composition.

Cosmetic compositions of the present invention can contain additional cationic polymers as conditioning agents. Suitable polymers are those of cationic polymers best known with their CTFA category name Polyquaternium. Typical examples of those Polyquaternium 6, Polyquaternium 7, Polyquaternium 10, Polyquaternium 11, Polyquaternium 16, Polyquaternium 22, Polyquaternium 28 and Polyquaternium 37.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84. It has been found out that Quaternium-80 is the best suitable one among the Quaterniums.

It has further been found out that especially those of cationic cellulose type polymers known as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The cationic polymers also include the quaternized products of graft polymers from organopolysiloxanes and polyethyl oxazolines described in EP-A 524 612 and EP-A 640 643.

The hair cosmetic compositions of the present invention can further comprise silicone-conditioning agents. Those can be volatile as well non volatile ones, as well as nonionic and cationic ones are found to be suitable. It should be noted that for the selection compatibility should be evaluated first. Typical examples of those suitable ones are of Dow Corning^{R} series, as well dimethicone, phenyldimethicone, cyclomethicone, polydimethylsiloxane, polydiethylsiloxane, polymethylphenyl siloxane are found to be suitable. As a cationic silicone derivative, amodimethicone is found to be effective.

Hair cosmetic compositions of the present invention can comprise additionally one or more cationic surfactants as conditioner presented with the general formula 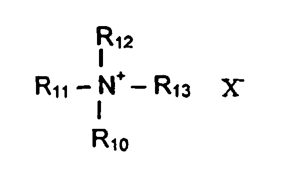 where R₁₀ is a saturated or unsaturated, branched or non-branched alkyl chain with 8-22 C atoms or

R₁₄ CO NH (CH₂)ₙ

where R₁₄ is saturated or unsaturated, branched or non-branched alkyl chain with 7-21 C atoms and n has value of 1 - 4,
or

R₁₅ CO O (CH₂)ₙ

where R₁₅ is saturated or unsaturated, branched or non-branched alkyl chain with 7-21 C atoms and n has value of 1 - 4, and
R₁₁ is H or unsaturated or saturated, branched or non-branched alkyl chain with 1 - 4 C atoms or

R₁₄ CO NH (CH₂)ₙ

or

R₁₅ CO O (CH₂)ₙ

where R₁₄, R₁₅ and n are same as above.

R₁₂ and R₁₃ are H or lower alkyl chain with 1 to 4 carbon atoms, and X is anion such as chloride, bromide, methosulfate.

Typical examples of those ingredients are cetyl trimethly ammonium chloride, stear trimonium chloride, behentrimoinium chloride, stearamidopropyl trimonuim chloride, dioleoylethyl dimethyl ammonium methosulfate, dioleoylethyl hydroxyethylmonium methosulfate.

Typical concentration range for any of those additional conditioners of cationic polymers, silicon oil and derivatives and cationic surfactants can be 0.01 - 10% by weight, preferably 0.01 - 5% by weight, more preferably 0.05 - 2% by weight calculated to the total composition.

Fatty acids are also found to be effective hair repair agents according to the invention. The preferred ones are C₁₂ to C₂₄ fatty acids with or without one or more unsaturation in the chain. The preferred concentration of fatty acids is in the range of 0.1 to 5%, preferably 0.1 to 3% by weight and more preferably 0.1 to 2.5% by weight calculated to the total composition.

Sterol like molecules especially of those plant originated phytosterols are as well found to be very suitable repair ingredients according to the invention. The most preferred is the one from plant origin avocadin, which is the unsaponifiable part of avocado oil. The others suitable ones as well listed in the application EP 1 036 556. The preferred concentration is in the range of 0.1 to 5%, preferably 0.1 to 3% by weight and more preferably 0.1 to 2.5% by weight calculated to the total composition.

Fatty alcohols are as well part of the compositions according to the invention. The preferred are according to the formula

R₁₆ - OH

where R₁₆ is an alkyl chain with 12 to 22 C atoms.

Concentration of fatty alcohols, usually present as a mixture, is very much dependent on the type of the preparations. In cleaning compositions the concentration is usually low around 1% or low, whereas in emulsion type of preparations, the concentration can be in the range of 1 to 15%, preferably 1-10% by weight calculated to the total composition. The most preferred fatty alcohol is mixture of cetyl and stearyl alcohols, better known as cetearyl alcohol. The mixing ration can be any from 3:7 to 7:3, cetyl to stearyl alcohols.

The compositions according to the invention may also comprise additional conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Further conditioning additives are hair conditioning and/or styling polymers. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in composition of the present invention. They are incorporated alone or in admixture with at least one additional cationic, nonionic or anionic polymer, particularly copolymers of N-octyl acrylamide, (meth)acrylic acid and tert.-butyl aminoethyl methacrylate of the type "Amphomer®"; copolymers from methacryl oylethyl betaine and alkyl methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl aminoalkyl (meth)acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199, are applicable.

Anionic polymers useful are vinyl alkyl ether, in particular methyl vinyl ether/maleic acid copolmers, obtained by hydrolysis of vinyl ether/maleic acid anhydride copolymers, distributed under the trade name "Gantrez® AN or ES". These polymers may also be partly esterified, for example, "Gantrez® ES 225", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g., "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/aryl amide copolymers or the sodium salts thereof of the type "Reten®"; etc.

Hair cosmetic composition of the present invention, especially shampoo compositions, can be transparent as well a pearly. Transparency of the composition is judged by naked eye in a transparent shampoo bottle with a thickness not more than 5 cm. In the case a transparent appearance is wished, the following ingredients are not essential. However, in the case of cleansing shampoo preparations, pearl-shiny appearance is achieved with those dispersed in shampoo compositions in crystalline form, i.e. so called pearl-shine agents. The preferred once are PEG-3 distearate and ethylene glycol distearate. The concentration of those can typically be from 0.1 to 3%, preferably 0.5 to 2% by weight, calculated to the total composition.

The pH of compositions according to the invention is in the range of 2 to 8, preferably 2 to 6, more preferably 2.5 to 6. For adjusting the pH of the said conditioning shampoo composition, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be chosen in a way that composition reaches the desired pH value as given above. Typically concentration for acids can be 0.01 - 3% by weight, preferably 0.05 - 2% by weight, more preferably 0.05 - 1% by weight calculated to the total composition. The pH of the composition can also be adjusted to the required pH by using alkaline solution such as sodium hydroxide, potassium hydroxide or their salts with those acids mentioned above in the case that at the selected acid concentration pH of the composition is lower than that of the aimed value. In the case that salts are used for adjusting pH, concentration of salts should not exceed 3% by weight.

The compositions of the present invention may contain organic solvents such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. Concentration of organic solvents in the shampoo composition should not exceed 5% by weight. The role of the organic solvent, especially in conditioner, treatment preparations, is penetration enhancing.

Solubilizers may be added to the compositions especially when oily substances are chosen as conditioning agents and fragrance oils with highly lipophilic properties. Typical solubilizers may be hydrogenated castor oil known with the trade mark Cremophor RH series from BASF. It should be noted that as well the surfactant mixture can be a good solubilizer for fragrance oils. Typical concentration of the solubilizers can be in the range of 0.01 - 2% by weight, preferably 0.1 - 1% by weight, in the case additional solubilizer used as mentioned above, other surfactants used for shampooing purposes.

The composition may contain active ingredients selected from UV filters, moisturisers, sequestering agents, antidandruff agents, and natural ingredients showing conditioning benefits.

Suitable UV filters are either oil soluble, nonionics, or water soluble ones, mainly anionic. The examples to oil soluble ones are octyl methoxy cinnamate, benzophenon-3, and to water soluble ones, those with sulphonic acid group carrying ones and the best-known example is that benzophenone-4.

The moisturising agents are selected from panthenol, polyols, such as glycerol, polyethylene glycols with molecular weight 200 to 20,000. The moisturising ingredients can be included in the conditioner compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

Antidandruff agent is preferably piroctone olamine (Octopirox). Other antidandruff agents such as climbazol can also be used as long as those are soluble in the conditioner compositions or can be solubilized with the aid of the solublizers mentioned above.

The viscosity of the hair cosmetic compositions according to the invention for shampoo and rinse off conditioners is in the range of 1,000 and about 50,000 mPa.s at 20°C, preferably 1,000 to 30,000, in particular 1,500 to 20,000 mPa.s at 20°C, and most preferred 2,000 to 15,000 measured with Brookfield or Höppler viscosimeters at a shear rate of 10 sec⁻¹. For leave in especially spray types the composition can be very thin in consistency, i.e. have viscosity lower than the most preferred lower range of 2000 mPa.s. It should also be noted that sprayable conditioners can as well be in gel form.

It is self-understood that the hair cosmetic compositions according to the invention may comprise all substances customarily used in such compositions.

Examples of such substances are complex formers, dyestuffs for coloring compositions mainly of anionic character, preservatives, viscosity regulants (thickening agents), such as inorganic salts, to the extent they are not already contained in the original surfactant mixtures, fragrances, moisture retainers, plant and animal oils, such as jojoba oil, etc. A list of such additives can also be found in Schrader, I.c., on pp. 695 to 722.

The following Examples illustrate the invention. The products according to the invention are prepared by mixing the individual components in water, whereby it is also possible to use pre-mixtures of various ingredients.

### Example 1

| **Shampoo** | |
|---|---|
| C₁₂-C₁₄-Fatty alcohol ether sulfate | 10.0 (% by wt.) |
| C₈-C₂₂-Alkyl glucoside (P.D.:~ 1.5) | 5.0 |
| Sodium lauroyl glutamate | 2.0 |
| Cocoamidopropyl betaine | 3.2 |
| Cetyl PEG/PPG-10/1 dimethicone | 0.5 |
| Ceramide of formula I* | 0.4 |
| Behenic acid | 0.5 |
| Avocadin | 0.25 |
| Cationic polymer (Polyquaternium-7) | 0.1 |
| Sodium benzoate | 0.6 |
| Sodium sorbate | 0.3 |
| Peach oil | 0.1 |
| Perfume | 0.7 |
| PEG-60-hydrogenated castor oil | 0.5 |
| Benzophenone-3 | 0.1 |
| Citric acid | q.s. pH 5.0 |
| Water | ad 100.0 |

| | |
|---|---|
| * R₁ is C₁₅H₃₁; R₂ is C₁₆H₃₃ and R₃ and R₄ are H and n=2 | |

This shampoo was compared in a known half-head double blind test on 10 test persons with a shampoo containing no cetyl PEG/PPG-10/1 dimethicone and ceramide according to the formulation.

The separate evaluation by two hairdressers resulted in a preference of 9:1 in favor of the shampoo according to the invention with regard to wet and dry combability, smoothness and elasticity of the wet hair, and of 8:2 in favor of the shampoo according to the invention with regard to volume, bounce, shine and smoothness and softness of the dried hair. The natural look of hair compared to the side washed with non-inventive compositions is as well reported by hair dressers as an advantage

### Example 2

| **Shampoo** | |
|---|---|
| Sodium lauryl ether sulfate | 10.0 (% by wt.) |
| Sodium lauroyl glutamate | 4.0 |
| C₁₂-C₁₄-alkyl glucoside (P.D.:~ 1.4) | 3.0 |
| Cocoamidopropyl betaine | 3.5 |
| Cetyl PEG/PPG-10/1 dimethicone | 0.7 |
| Ceramide of formula I* | 0.4 |
| Cationic polymer (Polyquaternium-11) | 0.2 |
| PEG-3 distearate | 0.8 |
| Perfume | 0.6 |
| Sodium benzoate | 0.6 |
| Benzophenone-4 | 0.2 |
| Citric acid | q.s. to pH 5.5 |
| Water | ad 100.0 |

| | |
|---|---|
| * R₁ is C₁₅H₃₁; R₂ is C₁₆H₃₃ and R₃ and R₄ are H and n=2 | |

A shampoo with very good lathering capability and hair conditioning properties was obtained.

### Example 3

| **Shampoo** | |
|---|---|
| Sodium lauryl ether carboxylate (10EO) | 5.0 (% by wt.) |
| Disodium lauryl ether sulfosuccinate | 3.5 |
| Decylglucoside (P.D.:~ 1.4) | 2.5 |
| Sodium lauroyl glutamate | 3.0 |
| Cocoamidopropyl betaine | 3.0 |
| Cetyl PEG/PPG-10/1 dimethicone | 0.5 |
| Ceramide of formula I* | 0.2 |
| Avocadin | 0.1 |
| Cetearyl alcohol | 1.0 |
| Cationic polymer (Polyquaternium-10) | 0.2 |
| Sodium benzoate | 0.6 |
| Citric acid | q.s. pH 5.0 |
| Perfume | 0.5 |
| Water | ad 100.0 |

| | |
|---|---|
| * R₁ is C₁₅H₃₁; R₂ is C₁₆H₃₃ and R₃ and R₄ are H and n=2 | |

The properties of this shampoo corresponded with those according to Examples 1 and 2.

### Example 4

| **Shampoo** | |
|---|---|
| Sodium lauryl ether sulfate | 10.5 (% by wt.) |
| Sodium lauroyl sarcosinate | 4.0 |
| Sodium lauroyl glutamate | 1.0 |
| C₁₂-C₁₄-alkyl glucoside (P.D.:~1.5) | 2.5 |
| Cocodimethyl amineoxide | 1.5 |
| Sodium cocoamidopropylbetaine | 2.5 |
| Cetyl PEG/PPG-10/1 dimethicone | 0.5 |
| Cetrimonium chloride | 0.5 |
| Ceramide of formula I* | 0.5 |
| Stearic acid | 0.3 |
| Preservative | 0.5 |
| Citric acid | 0.5 |
| Lactic acid | 0.1 |
| Pyrrolidone carboxylic acid | 0.1 |
| Glycolic acid | 0.1 |
| Malic acid | 0.1 |
| Perfume | 0.5 |
| Benzophenone-3 | 0.3 |
| Water | ad 100.0 |

| | |
|---|---|
| * R₁ is C₁₅H₃₁; R₂ is C₁₆H₃₃ and R₃ and R₄ are H and n=2 | |

pH of the shampoo is 4.5. This shampoo provides the hair with gloss, volume and excellent wet and dry combability as well good styling properties are observed.

### Example 5

| **Shampoo** | |
|---|---|
| Sodium lauryl ether sulfate | 10.0 (% by wt.) |
| Sodium lauroyl glutamate | 4.0 |
| C₁₂-C₁₄-alkyl glucoside (P.D.:~ 1.4) | 3.0 |
| Cocoamidopropyl betaine | 3.5 |
| Cetyl PEG/PPG-10/1 dimethicone | 0.7 |
| Ceramide of formula I* | 0.5 |
| Stearic acid | 0.3 |
| Avocadin | 0.1 |
| Dimethicone | 0.5 |
| PEG-3 distearate | 0.8 |
| Perfume | 0.6 |
| PEG 160 hydrogenated castor oil | 0.6 |
| Sodium benzoate | 0.6 |
| Citric acid | q.s. pH 5.5 |
| Water | ad 100.0 |

| | |
|---|---|
| * R₁ is C₁₅H₃₁; R₂ is C₁₆H₃₃ and R₃ and R₄ are H and n=2 | |

This shampoo provides the hair with gloss, volume and excellent wet and dry combability.

### Example 6

| **Shampoo** | |
|---|---|
| Sodium lauryl ether sulfate | 10.0 (% by wt.) |
| Sodium lauroyl glutamate | 4.0 |
| C₁₂-C₁₄-alkyl glucoside (P.D.:~ 1.4) | 3.0 |
| Cocoamidopropyl betaine | 3.5 |
| Cetyl PEG/PPG-10/1 dimethicone | 0.7 |
| Dimethicone | 0.5 |
| Ceramide of formula I* | 0.5 |
| Stearic acid | 0.3 |
| Cetearyl alcohol | 0.7 |
| Panthenol | 1.0 |
| PEG-3 distearate | 0.8 |
| Perfume | 0.6 |
| PEG 160 hydrogenated castor oil | 0.6 |
| Sodium benzoate | 0.6 |
| Benzophenone-4 | 0.1 |
| Citric acid | q.s. pH 5.5 |
| Water | ad 100.0 |

| | |
|---|---|
| * R₁ is C₁₅H₃₁; R₂ is C₁₆H₃₃ and R₃ and R₄ are H and n=2 | |

This shampoo is found to be suitable especially for dry hair and provides the hair with gloss, volume and excellent wet and dry combability. Hair feels excellently soft after drying.

### Example 7

| **Rinse off conditioner** | |
|---|---|
| Cetearyl alcohol | 5.0 (% by wt.) |
| DiC12 - C15 alkyl dimethyl ammonium chloride | 1.0 |
| C₁₂-C₁₄-alkyl glucoside (P.D.:~ 1.4) | 1.0 |
| Cetrimonium chloride | 1.0 |
| Cetyl PEG/PPG-10/1 dimethicone | 0.7 |
| Dimethicone | 0.5 |
| Ceramide of formula I* | 0.5 |
| Stearic acid | 0.3 |
| Panthenol | 1.0 |
| Benzyl glycol | 3.0 |
| Perfume | 0.6 |
| Sodium benzoate | 0.6 |
| Citric acid | q.s pH 4.5 |
| Water | ad 100.0 |

| | |
|---|---|
| * R₁ is C₁₅H₃₁; R₂ is C₁₆H₃₃ and R₃ and R₄ are H and n=2 | |

In a half side test, inventive composition against a composition without ceramide, Cetyl PEG/PPG-10/1 dimethicone showed very favourable results (8 to 2) for inventive compositions in terms of volume, elasiticity, smoothness, lightness, softness and especially shine in dry stage.

### Example 8

| **Spray conditioner** | |
|---|---|
| Ethanol | 45.0 (% by wt.) |
| Polyquaternium 6 | 0.1 |
| Cetyl PEG/PPG-10/1 dimethicone | 0.1 |
| Ceramide of formula I* | 0.1 |
| Stearic acid | 0.1 |
| Panthenol | 1.0 |
| PEG 160 hydrogenated castor oil | 0.2 |
| Perfume | 0.1 |
| Citric acid | 0.1 |
| Water | ad 100.0 |

| | |
|---|---|
| * R₁ is C₁₅H₃₁; R₂ is C₁₆H₃₃ and R₃ and R₄ are H and n=2 | |

The composition is applicable with a pump spray on shampooed hair. The results showed that shiny, loose, voluminous and soft hair is obtained after treatment with the above composition.

### Example 8

| **Rinse off conditioner** | |
|---|---|
| Cetearyl alcohol | 5.0 (% by wt.) |
| Dioleoylethyl hydroxyethylmonium methosulfate | 1.0 |
| Ceteareth-16 | 0.8 |
| Cetyl PEG/PPG-10/1 dimethicone | 0.7 |
| Ceramide of formula I* | 0.5 |
| Stearic acid | 0.3 |
| Avocadin | 0.5 |
| Panthenol | 1.0 |
| Benzyl glycol | 3.0 |
| Perfume | 0.6 |
| Sodium benzoate | 0.5 |
| Benzophenone-3 | 0.3 |
| Citric acid | q.s pH 3.5 |
| Water | ad 100.0 |

| | |
|---|---|
| * R₁ is C₁₅H₃₁; R₂ is C₁₆H₃₃ and R₃ and R₄ are H and n=2 | |

The composition is applied as a intensive conditioner for hair repair purpose on a strongly damaged hair. After application a processing time is allowed for 10 min under heat at around 40°C. The results showed that shiny, loose, voluminous and soft hair is obtained after treatment with the above composition. Hair dressers as well commented the hair to be feeling healthier.

### Example 9

| **Leave-in conditioner** | |
|---|---|
| Cetearyl alcohol | 5.0 (% by wt.) |
| Stearamidopropylamine | 1.0 |
| Ceteareth-16 | 0.3 |
| Cetyl PEG/PPG-10/1 dimethicone | 0.7 |
| Ceramide of formula I* | 0.5 |
| Stearic acid | 0.3 |
| Avocadin | 0.5 |
| Panthenol | 1.0 |
| Ethanol | 5.0 |
| Perfume | 0.6 |
| Sodium benzoate | 0.5 |
| Benzophenone-3 | 0.3 |
| Citric acid | q.s pH 4.0 |
| Water | ad 100.0 |

| | |
|---|---|
| * R₁ is C₁₅H₃₁; R₂ is C₁₆H₃₃ and R₃ and R₄ are H and n=2 | |

The composition is applied as a leave-in conditioner for hair repair purpose on a strongly damaged hair after shampoing. The results showed that shiny, loose, voluminous and soft hair is obtained after treatment with the above composition. Hair dressers as well commented the hair to be feeling healthier.

## Claims

1. Aqueous cosmetic composition for hair **characterized in that**, it comprises cetyl PEG/PPG-10/1 dimethicone and at least one ceramide type of ingredient of the following formula 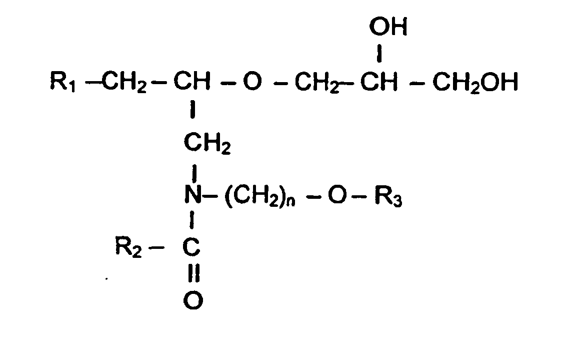 where R¹ and R² are independent from each other alkyl- or alkenyl group mit 10 to 22 carbon atoms, R³ is methyl, ethyl, n-propyl or isopropyl group and n is a number between 1 to 6, preferably 2 or 3.

2. Aqueous cosmetic composition according to claim 1 **characterized in that**, it is an aqueous shampoo composition and comprises additional at least one foaming surfactant selected form anionic, nonionic and amphoiteric and zwitterionic ones.

3. Aqueous shampoo composition according to claim 2 **characterized in that** concentration of foaming surfactants is in the range of 1 to 50% by weight calculated to the total composition.

4. Aqueous shampoo composition according to claim 2 **characterized in that** the ratio between the anionic main surfactant and the nonionic and amphoteric or zwitterionic co-surfactants is in the range of 10:1 to 1:3.

5. Aqueous shampoo composition according to claim 2 **characterized in that** the ratio between the anionic main surfactant and the nonionic or amphoteric or zwitterionic co-surfactant is in the range of 10:1 to 1:1.

6. Aqueous shampoo composition according to claim 2 **characterized in that** it is a non-transparent pearly composition and contains perlizing agents at a concentration of 0.1 to 3% by weight calculated to total composition.

7. Aqueous cosmetic composition according to claim 1 **characterized in that**, it is a rinse off conditioner.

8. Aqueous cosmetic composition according to claim 1 **characterized in that**, it is a leave in conditioner.

9. Aqueous cosmetic composition according to any of the preceding claims **characterized in that**, it comprises fatty alcohol or their mixtures.

10. Aqueous cosmetic composition according to any of the preceding claims **characterized in that**, that it comprises as an additional ingredient fatty acids at a concentration of 0.1 to 5% by weight calculated to total composition.

11. Aqueous cosmetic composition according to any of the preceding claims **characterized in that**, that it comprises as an additional ingredient phytosterol at a concentration of 0.1 to 5% by weight calculated to total composition.

12. Aqueous cosmetic composition according to any of the preceding claims **characterized in that** it has a pH in the range of 2 to 8.

13. Aqueous conditioning shampoo composition according to any of the preceding claims **characterized in that** it comprises additional conditioning agents selected from cationic polymers, cationic surfactants and silicone oils or siloxanes.

14. Aqueous conditioning shampoo composition according to any of the preceding claims **characterized in that** it contains organic solvents at a concentration not exceeding 5% by weight calculated to the total composition.

15. Aqueous conditioning shampoo composition according to any of the preceding claims **characterized in that** it contains active ingredients selected from UV filter, moisturisers, sequestering agents, antidandruff agents, and natural ingredients showing conditioning benefits.

## Patentansprüche

1. Wässrige, kosmetische Zusammensetzung zur Behandlung von Haaren, **dadurch gekennzeichnet, dass** sie Cetyl-PEG/PPG-10/1 dimethicone und mindestens einen Ceramide-Typen mit der Strukturformel 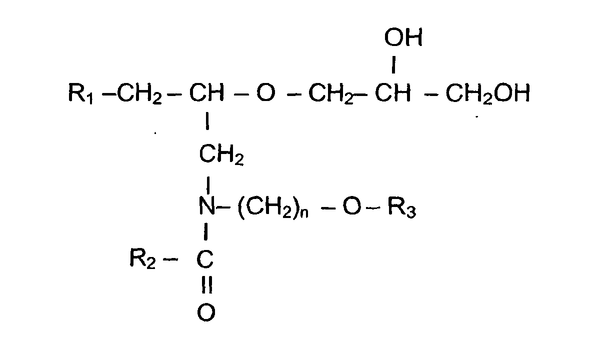 enthält, worin R₁ und R₂ unabhängig voneinander eine Alkyl- oder Alkenylgruppe mit 10 bis 22 Kohlenstoffatomen, R₃ Methyl, Ethyl, n-Propyl oder Isopropyl und n eine Zahl zwischen1 bis 6, vorzugsweise 2 oder 3, ist.

2. Wässrige, kosmetische Zusammensetzung nach Anspruch 1, **dadurch gekennzeichnet, dass** es ein wasserhaltiges Shampoo ist, welches zusätzlich mindestens ein schäumendes Tensid, ausgewählt aus anionischen, nichtionischen, amphoterischen und zwitterionischen Tensiden, enthält.

3. Wässrige Shampoo-Zusammensetzung nach Anspruch 2, **dadurch gekennzeichnet, dass** das schäumende Tensid in einer Menge von 1 bis 50 Gew.-%, berechnet auf die Gesamtzusammensetzung, enthalten ist.

4. Wässrige Shampoo-Zusammensetzung nach Anspruch 2, **dadurch gekennzeichnet, dass** das Verhältnis zwischen dem anionischen Haupttensid und den nichtionischen und amphoterischen oder zwitterionischen Co-Tensiden im Bereich von 10:1 bis 1:3 liegt.

5. Wässrige Shampoo-Zusammensetzung nach Anspruch 2, **dadurch gekennzeichnet, dass** das Verhältnis zwischen dem anionischen Hauttensid und den nichtionischen oder amphoterischen oder zwitterionischen Co-Tensiden im Bereich von 10:1 bis 1:1 liegt.

6. Wässrige Shampoo-Zusammensetzung nach Anspruch 2, **dadurch gekennzeichnet, dass** es eine nichttransparente, perlglanzhaltige Zusammensetzung ist, welche Perlglanzmittel in einer Menge von 0,1 bis 3 Gew.-% , berechnet auf die Gesamtzusammensetzung, enthält.

7. Wässrige, kosmetische Zusammensetzung nach Anspruch 1, **dadurch gekennzeichnet, dass** es ein "rinse off Conditioner" ist.

8. Wässrige, kosmetische Zusammensetzung nach Anspruch 1, **dadurch gekennzeichnet, dass** es ein "leave in Conditioner" ist.

9. Wässrige, kosmetische Zusammensetzung nach einem der Ansprüche 1 bis 8, **dadurch gekennzeichnet, dass** sie Fettalkohole oder Fettalkoholmischungen enthält.

10. Wässrige, kosmetische Zusammensetzung nach einem der Ansprüche 1 bis 9, **dadurch gekennzeichnet, dass** sie zusätzlich Fettsäuren in einer Menge von 1 bis 5 Gew.-%, berechnet auf die Gesamtzusammensetzung, enthält.

11. Wässrige, kosmetische Zusammensetzung nach einem der Ansprüche 1 bis 10, **dadurch gekennzeichnet, dass** sie zusätzlich Phytosterol in einer Menge von 0,1 bis 5 Gew.-%, berechnet auf die Gesamtzusammensetzung, enthält.

12. Wässrige, kosmetische Zusammensetzung nach einem der Ansprüche 1 bis 11, **dadurch gekennzeichnet, dass** sie eine pH-Wert von 2 bis 8 aufweist.

13. Wässrige Pflegeshampoo-Zusammensetzung nach einem der Ansprüche 1 bis 12, **dadurch gekennzeichnet, dass** sie zusätzlich eine Pflegesubstanz, ausgewählt aus kationischen Polymeren, kationischen Tensiden und Silikonölen oder Siloxanen, enthält.

14. Wässrige Pflegeshampoo-Zusammensetzung nach einem der Ansprüche 1 bis 13, **dadurch gekennzeichnet, dass** sie organische Lösungsmittel in einer Menge von maximal 5 Gew.-%, berechnet auf die Gesamtzusammensetzung, enthält.

15. Wässrige Pflegeshampoo-Zusammensetzung nach einem der Ansprüche 1 bis 14, **dadurch gekennzeichnet, dass** sie aktive Wirksubstanzen, ausgewählt aus UV-Filtern, Feuchtigkeitsspendern, Komplexbildnern, Antischuppenmitteln und natürlichen Substanzen mit Pflegeeffekt, enthält.

## Revendications

1. Composition cosmétique aqueuse pour les cheveux, **caractérisée en ce qu'**elle comprend de la cétyl-PEG/PPG-10/1 diméthicone et au moins un ingrédient de type de céramide de formule suivante 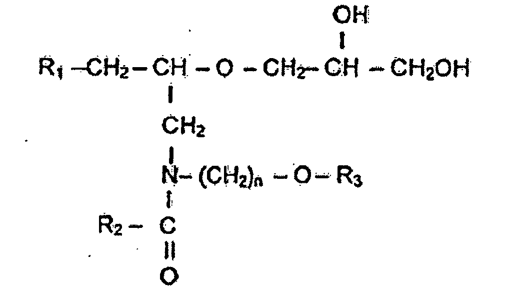 dans laquelle R¹ et R² sont indépendamment l'un de l'autre un groupe alkyle ou alcényle comportant 10 à 22 atomes de carbone, R³ est un groupe méthyle, éthyle, n-propyle ou isopropyle et n est un nombre de 1 à 6, de préférence 2 ou 3.

2. Composition cosmétique aqueuse selon la revendication 1, **caractérisée en ce qu'**elle est une composition aqueuse de shampoing et qu'elle comprend de plus au moins un tensioactif moussant choisi parmi les tensioactifs anioniques, non ioniques, amphotères et zwittérioniques.

3. Composition aqueuse de shampoing selon la revendication 2, **caractérisée en ce que** la concentration des tensioactifs moussants est dans la gamme de 1 à 50 % en poids, rapportée à la composition totale.

4. Composition aqueuse de shampoing selon la revendication 2, **caractérisée en ce que** le rapport entre le tensioactif anionique principal et les co-tensioactifs non ioniques et amphotères ou zwittérioniques est dans la gamme de 10:1 à 1:3.

5. Composition aqueuse de shampoing selon la revendication 2, **caractérisée en ce que** le rapport entre le tensioactif anionique principal et les co-tensioactifs non ioniques ou amphotères ou zwittérioniques est dans la gamme de 10:1 à 1:1.

6. Composition aqueuse de shampoing selon la revendication 2, **caractérisée en ce qu'**elle est une composition nacrée non transparente et qu'elle contient des agents nacrants en une concentration de 0,1 à 3 % en poids, rapportée à la composition totale.

7. Composition cosmétique aqueuse selon la revendication 1, **caractérisée en ce qu'**elle est une composition conditionneur avec rinçage.

8. Composition cosmétique aqueuse selon la revendication 1, **caractérisée en ce qu'**elle est une composition conditionneur sans rinçage

9. Composition cosmétique aqueuse selon l'une quelconque des revendications précédentes, **caractérisée en ce qu'**elle comprend un alcool gras ou leurs mélanges.

10. Composition cosmétique aqueuse selon l'une quelconque des revendications précédentes, **caractérisée en ce qu'**elle comprend, en tant qu'ingrédient supplémentaire, des acides gras en une concentration de 0,1 à 5 % en poids, rapportée à la composition totale.

11. Composition cosmétique aqueuse selon l'une quelconque des revendications précédentes, **caractérisée en ce qu'**elle comprend, en tant qu'ingrédient supplémentaire, du phytostérol en une concentration de 0,1 à 5 % en poids, rapportée à la composition totale.

12. Composition cosmétique aqueuse selon l'une quelconque des revendications précédentes, **caractérisée en ce qu'**elle a un pH dans la gamme de 2 à 8.

13. Composition aqueuse de type shampoing conditionneur selon l'une quelconque des revendications précédentes, **caractérisée en ce qu'**elle comprend des agents de conditionnement supplémentaires choisis parmi les polymères cationiques, les tensioactifs cationiques et les huiles de silicone ou les siloxanes.

14. Composition aqueuse de type shampoing conditionneur selon l'une quelconque des revendications précédentes, **caractérisée en ce qu'**elle contient des solvants organiques en une concentration ne dépassant pas 5 % en poids, rapportée à la composition totale.

15. Composition aqueuse de type shampoing conditionneur selon l'une quelconque des revendications précédentes, **caractérisée en ce qu'**elle contient des ingrédients actifs choisis parmi un filtre UV, des humidifiants, des agents séquestrants, des agents antipelliculaire et des ingrédients naturels présentant des avantages sur le plan du conditionnement.
